Europäisches Patentamt

European Patent Office

Office européen des brevets

(11) Publication number: **0 231 969 B1**

# EUROPEAN PATENT SPECIFICATION

(45) Date of publication of patent specification: **19.08.92**  (51) Int. Cl.⁵: **A61B 6/00,** A61B 6/10

(21) Application number: **87200110.2**

(22) Date of filing: **26.01.87**

The file contains technical information submitted after the application was filed and not included in this specification

(54) **Mobile X-ray diagnostic apparatus.**

(30) Priority: **31.01.86 NL 8600227**

(43) Date of publication of application:
**12.08.87 Bulletin 87/33**

(45) Publication of the grant of the patent:
**19.08.92 Bulletin 92/34**

(84) Designated Contracting States:
**DE FR GB IT NL**

(56) References cited:
**EP-A- 0 068 931       EP-A- 0 109 206
CH-A- 213 139        DE-A- 1 939 557
DE-A- 3 439 968      DE-B- 1 213 565
US-A- 3 549 885**

(73) Proprietor: **N.V. Philips' Gloeilampenfabrieken
Groenewoudseweg 1
NL-5621 BA Eindhoven(NL)**

(72) Inventor: **Cobben, Johannes Ignatius Marie
c/o INT. OCTROOIBUREAU B.V. Prof. Holst-
laan 6
NL-5656 AA Eindhoven(NL)**

(74) Representative: **Scheele, Edial François et al
INTERNATIONAAL OCTROOIBUREAU B.V.
Prof. Holstlaan 6
NL-5656 AA Eindhoven(NL)**

## Description

The invention relates to an X-ray diagnostic apparatus comprising a mobile support having a number of wheels, a C-arc carried by the support, an X-ray source and an image intensifier tube attached to respective ends of said C-arc.

To ensure good image formation in X-ray diagnostic apparatus of this type it is very important for the X-ray source and the X-ray image intensifier tube to occupy an accurately reproducible, stable position with respect to each other. Normally, in order to position the apparatus with respect to, for example, a patient to be examined, the entire apparatus is simply placed in a desired position with respect to the latter, for which purpose the apparatus is mobile in construction, and the combination of source and detector is adjusted by moving the C-arc in the support. For this purpose, the C-arc is mounted in the support in such a way that it can be rotated around an axis through the mid-point (isocentre) of the C-arc, its height can be adjusted with respect to the support and it can be shifted along the circumference of the C-arc.

Document CH-A-213 139 discloses a transportable X-ray system having a C-arc; the X-ray source and X-ray detector of said system are interchangeable at each end of said C-arc.

For special applications it is desirable for the apparatus to be capable of being divided into easily manageable parts in order to permit it to be transported easily and with little possibility of damage between various locations where it is used. Here the dilemma arises that a division into several small parts is advantageous for ensuring safe transport, but that the stability and exact position of the components in mounted form can be negatively affected as a result. Division into larger parts has the disadvantage that transport becomes less safe or much more time-consuming because units which are still assembled must be packed as a whole, completely shock-free. The most significant part of the apparatus in this respect is the C-arc with the X-ray source and the image intensifier tube. Both an X-ray tube with the related power supply, diaphragm etc. and an X-ray image intensifier tube with screening, television camera tube, power supply, etc. are relatively heavy components. In addition, it is precisely these two components which are very sensitive to shocks and therefore require good shock-free packaging. The C-arc as such is much less sensitive to shocks and a much less strictly shock-free packaging can suffice for it. This is all the more important because of the special shape and the relatively large dimensions of the C-arc. The disadvantages mentioned are further intensified by the fact that a unit which comprises the C-arc, the X-ray tube and the image intensifier tube is extra vulnerable precisely in that combination when separate from the support, so that the danger of distortion of the C-arc during transport occurs. As a result, good reciprocal positioning later on may be made difficult or impossible. In the assembled state electrical contacts are usually established between the support or a supply unit and the C-arc by means of a composite flexible cable. It is, of course, relatively easy to disconnect a flexible cable of this type. On the other hand, connection of electrical contacts together with mechanical connections between the C-arc and the X-ray source or the X-ray image intensifier, respectively, is complicated.

The object of the invention is to provide an X-ray diagnostic apparatus that is easily transportable and can be accurately and easily be put into an operational mode.

Thereto, an X-ray diagnostic apparatus according to the invention is characterized in that the apparatus is composed of detachable modules comprising an electrical supply module, an X-ray source module and an image intensifier module, the X-ray source module and the image intensifier module being detachably connected to the C-arc through respective couplings, the couplings each comprising an electrical connector that upon connection of the X-ray source module and the image intensifier module to the C-arc electrically connect these modules to the electrical supply module.

Since in an apparatus according to the invention electrical connectors are fitted between the C-arc and an X-ray tube module and an X-ray image intensifier tube module connecting these modules to the electrical supply unit, these components can be easily dismantled for transport in compact units and each can be separately packed to ensure optimum freedom from shock. The empty C-arc module, which is not very vulnerable, can be packaged for transport relatively easy and is also readily manageable as such.

In a preferred embodiment the couplings are equipped with pilot pins for positioning the image intensifier module and the X-ray source module with respect to the C-arc. For the mechanical positioning, end faces of the C-arc can act as reference stop surfaces. In a further embodiment the electrical connectors comprise plugs and sockets, the plugs or sockets during positioning being displaceable with respect to the part to which they are attached, a spring being connected to the plugs or the sockets for exerting a force on the plugs and the sockets upon displacement. In order to avoid the need for high voltage connections at that point, in a preferred embodiment the high voltage supply for both an X-ray tube and for an X-ray image intensifier tube are incorporated in the relevant module.

An C-arc module in a preferred embodiment comprises the actual C-arc and a roller mechanism for the above-mentioned circular movement of the C-arc in the appratus. In addition, this module is detachable connected to a pedestal for the C-arc, for example, with a blockable dovetail joint. The pedestal together with a rotatable and height-adjustable column is then, in turn, mounted detachably in a support as a separate module.

In an embodiment, an electrical control and operating module is detachably coupled to the mobile support. In addition, a drive motor for C-arc movements connected to the support can be designed for manual operation when the electrical module is removed so that in this situation too the C-arc can be moved without an electrical power supply.

In a preferred embodiment an X-ray source module can be rotated around an axis across the end face of the C-arc at that point. As a result, the X-ray tube can also be used for exposures which are not recorded with the X-ray image intensifier tube on the C-arc but, for example, by a bucky positioned laterally for X-ray film exposures. For preference, rotating the X-ray tube module out of a position in which it is aimed at the X-ray image intensifier tube blocks the fluoroscopy mode of operation of the apparatus.

In a further preferred embodiment a leg of the support projecting in the direction of the mounted C-arc is fixed to a column section of the support so that it can be swung up. In addition, for preference care is taken to ensure that in that operation a fixed relation is not lost between control grips on the support and a control wheel in the swing-up leg. This is achieved in a preferred embodiment by blocking the coupling between the control grip and control wheel against changes when the leg is being swung up. The blocking cannot be cancelled until the leg is again in the working position. In another embodiment a column section of the support can be detached as a separate module and the above-mentioned leg section is mounted on the undercarriage so that it can be swung back completely. The blocking for the control mechanism can be maintained here, but alternatively the control can be taken over by other wheels.

In a preferred embodiment, activation of the apparatus is automatically blocked as long as the apparatus is not assembled completely correctly. To permit a logical assembly of the apparatus, the relevant modules can be provided with consecutive serial numbers. For preference, all the control elements to be assembled are finished in an alarm colour so that any decoupling in the operating phase is avoided. In addition, the colour of the control elements to be assembled and the above-mentioned serial numbers are, for preference, made to match each other.

On the basis of the drawing, some preferred embodiments according to the invention will be described in greater detail below. In the drawing:

Fig. 1 shows an X-ray diagnostic apparatus according to the invention in assembled form,

Fig. 2 shows an overview of reciprocally detachable modules of this and

Fig. 3 shows an example of packaged modules of this.

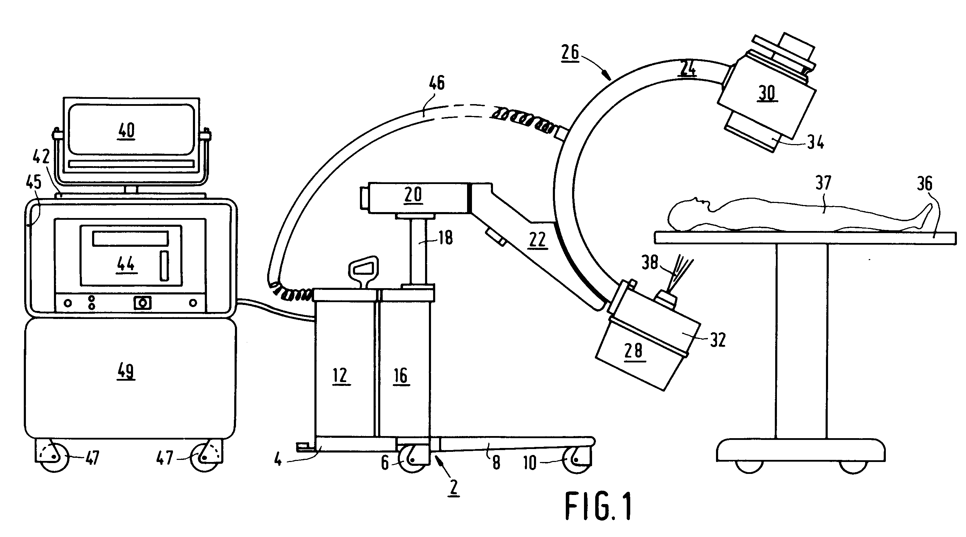

An X-ray diagnostic apparatus as drawn in Fig. 1 comprises a mobile support 2, with, here, a mobile undercarriage 4 with casters 6. The undercarriage 4 comprises a leg 8 with a caster 10. An electrical module 12 containing electrical power supplies, controls and a control panel is placed on the support 2. The support 2 comprises a column-shaped section 16 in which a pedestal 20 is incorporated via a cylinder 18. The pedestal 20 is connected to a C-arc 24 via a roller mechanism 22. The C-arc can rotate around the cylinder 18. By moving the cylinder 18 in the column-shaped section of the support the C-arc can be moved vertically, while the C-arc can be moved along an arc of circle 26 of the C-arc in the roller mechanism 22. An X-ray source module 28 and an X-ray image intensifier tube module 30 are coupled to the C-arc 24. The above-mentioned possible movements of the C-arc permit an X-ray tube 32 incorporated in the X-ray tube module and an X-ray image intensifier tube 34 placed in the X-ray image intensifier tube module 30 to be positioned with respect to a patient 37 lying on a supporting table 36. In this way, a patient can be irradiated from many directions with an X-ray beam 38 emitted by the X-ray source 32, while the X-ray beam, after passing the patient, is always intercepted by the X-ray image intensifier tube. For preference, the image intensifier tube module contains a television camera tube for reading out the image formed on an output screen of the image intensifier tube. A television monitor 40 with a control unit 42 is incorporated for reproducing the images recorded in this way and an image processing unit 44 is incorporated for image processing, image recording, image storage etc., which can, if necessary, also be equipped with a digital image processing system with a digital memory. A digital image processing system of this kind can, for example, also be equipped for forming digital subtraction images, for the reproduction of which a second monitor (not shown) can be incorporated if necessary. A flexible cable 46 ensures the electrical connection between the C-arc and the television playback or video processing system, preferably via the electrical power supply and control module 12, hereinafter called the electrical module. As is also shown in the figure, the image processing unit 44 is placed in a holder 45 in which it can also be transported and the monitor 40 is placed on a holder section 49 fitted with wheels 47 for the image processing unit. An image playback system fitted up in this way can also be used as a stand-alone unit, for example, for image playback from elsewhere or of image recordings made previously.

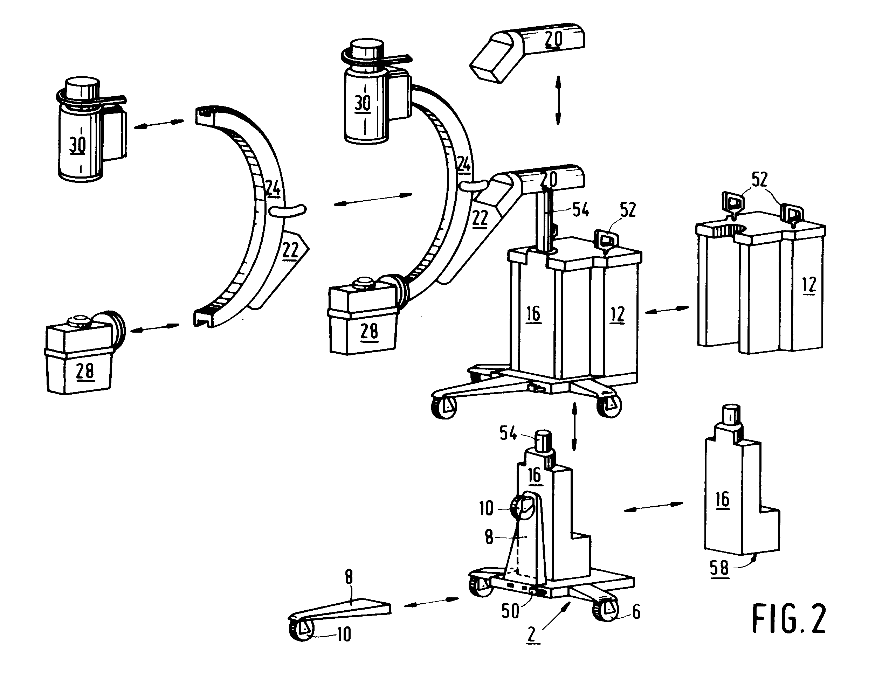

An overview of the various modules into which the image forming apparatus can be divided is presented in figure 2 and comprises the support module 2 with the undercarriage 4, casters 6 and leg 8 which is also shown here in the swung-up position. A lever 50 operates both an uncoupling mechanism whereby the leg 8 can be swung up, swung round or detached, and a coupling mechanism with which a given relation between the control handgrips 52 and the control wheel 10 is blocked. The blocking of the control device is only cancelled when the leg 8 is fixed in the operating position with the lever 50. Since the handgrips here are incorporated in the electrical module 12, coupling pins connected to the handgrips are incorporated in this which, for example, operate a chain transmission system between the handgrips 52 and the control wheel 10. The column section 16 of the support comprises a cylinder 54 and a drive motor, not shown here, for moving the C-arc with respect to the support. The drive mechanism for the C-arc in the dismantled state can be operated with a so-called agadir control in emergencies.

In another embodiment the column section 16 constitutes a separate column module. For preference, the leg 8 can then be swung round through 180° so that in the transport position this lies on the other section of the now separate undercarriage, but can also be mounted so that it is completely detachable. The control device for mobility can also be designed in such a way that the control function of the wheel 10 is taken over by the other two wheels of the support. The relatively complex coupling and blocking for swinging up the leg is thus avoided. A coupling does, however, remain between the handgrips 52 and, for example, a chain control drive for the wheels 6. The electrical module 12 can be coupled to a side of the carrier turned away from the leg 8, to which - as already mentioned - a coupling can also be made between the handgrips and the drive mechanism for the control wheel 10. All the coupling elements of the electrical module are mounted retracted with respect to an underside 58 of the module so that the electrical module can be put down without any danger of damage in the dismantled state.

The pedestal module 20 can be coupled to the support via the cylinder 54. In view of the favourable direction of gravity, the coupling can be carried out relatively easily and contains a clamping device to ensure a firm connection between the pedestal and support. The pedestal can be adjusted vertically via the cylinder 54 with the aid of the above-mentioned drive motor and the pedestal can also be rotated around the cylinder 54. The C-arc module, of which the shift mechanism 22 forms part, can be coupled to the pedestal, preferably by means of a dovetail joint which can be tightly clamped. The actual C-arc can perform a circular movement via the sliding mechanism. For preference, this movement mechanism is blocked when the connection between the pedestal and C-arc module is uncoupled, as a result of which the sliding mechanism takes up a fixed position with respect to the C-arc in the dismantled phase. As already mentioned, the C-arc with X-ray tube and X-ray image intensifier tube is a relatively unwieldy unit. In this unit the X-ray tube is incorporated in an X-ray tube module 28 which can be detached from the C-arc and the X-ray image intensifier tube is incorporated in a detector module 30. Strict positioning applies with regard to the coupling of these two modules, for which purpose, for example, the end faces of the C-arc can be shaped as a reference stop face. By means of pilot pins and a clamping device each of the modules can then easily be assembled in an exact position, for which purpose an auxiliary catch, for example, can be used. In addition, for the necessary electrical connections use is made of composite sockets which are positioned opposite each other by the mechanical pilot pins. Before forming the electrical contacts, repositioning is achieved here by fitting the elements which make mutual contact with an extra pilot pin construction. In addition, at least one of the contacting elements, preferably spring-mounted laterally, is fitted displaceably in the module. As a result, the plugs and sockets cannot be damaged during assembly. Here, too, care has been taken to ensure that the more vulnerable components are fitted recessed to some extent with respect to an outermost limit of the module at the relevant point. As an extra safety measure during transport, the contacting ends of the C-arc and its two modules which have to be coupled can be fitted with snap-on protective covers. For preference, all the decoupling mechanisms are designed in such a way that two deliberate successive operations must be performed to effect decoupling, for preference, a decoupling of a blocking of the mechanism for the actual decoupling. In the case of all the relevant decouplings, hence also for example in the case of an uncoupled image playback unit, the apparatus is blocked against operating during the fluoroscopy phase, as a result of which the emission of non-useful X-radiation is largely avoided. This naturally also applies for a construction of the apparatus geared to the exposure phase, such as the rotation of the X-ray tube module out of a position aimed at the image intensifier, the attachment of a light-beam pointer, and so on.

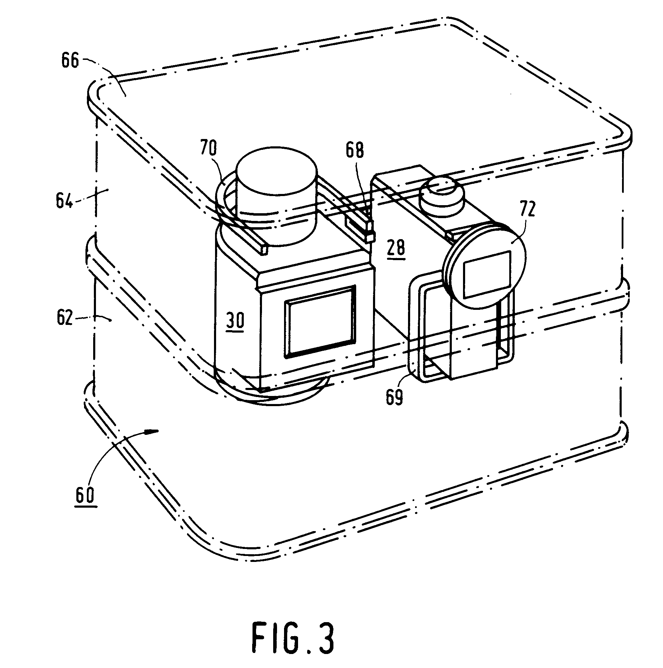

By way of example, figure 3 shows the X-ray source module 28 and the X-ray detector module 30 incorporated in a box 60 which consists here of parts 62 and 64 and a lid section 66. After removal of the lid section with a part of the shock-absorbent material, not shown in the drawing, which for example can also be permanently fixed to the lid section, the modules 28 and 30 are accessible but are still well protected in their storage place. By removing section 64 of the box the modules can be removed without lifting them up, partly for which purpose the modules are fitted with handgrips 68, 69 and 70. The X-ray source module here comprises a diaphragm holder 72. Other small components can be stored in recesses in the box beside the two modules in which respect, of course, the shock-proofing of the modules must not be impaired. In addition to the above-mentioned packaging for the image playback device as shown in fig. 1 and the box for the modules 28 and 30 shown in fig. 3, the whole apparatus can, for example, go into another box for the undercarriage module 4, a box for the column module 16, a box for the power supply and control module 12, and a box for the C-arc with, for example, the slide block assembly 20 and 22. For preference, the packaging for the image playback device 64 comprises two boxes which, as mentioned, are stacked one on top of the other in the operating phase. By advantageously selecting the dimensions, the external length, width and height of the various boxes, these can be transported completely, or almost completely, stacked in a closed block, in which respect it is nevertheless preferable for all the boxes to stand upright.

## Claims

1. X-ray diagnostic apparatus comprising a mobile support (2) having a number of wheels (6, 10), a C-arc (24) carried by the support, an X-ray source (32) and an image intensifier tube (34) attached to respective ends of said C-arc (24), characterized in that the apparatus is composed of detachable modules comprising an electrical supply module (12), an X-ray source module (28) and an image intensifier module (30), the X-ray source module (28) and the image intensifier module (30) being detachably connected to the C-arc (24) through respective couplings, the couplings each comprising an electrical connector that upon mechanical connection of the X-ray source module (28) and the image intensifier module (30) to the C-arc (24) electrically connects these modules to the electrical supply module (12).

2. X-ray diagnostic apparatus according to Claim 1, characterized in that the couplings are equipped with pilot pins for positioning the image intensifier module (30) and the X-ray source module (28) with respect to the C-arc (24).

3. X-ray diagnostic apparatus according to Claim 2, characterized in that the electrical connectors comprise plugs and sockets, the plugs or sockets during positioning being displaceable with respect to the part to which they are attached, a spring being connected to the plugs or the sockets for exerting a force on the plugs and the sockets upon displacement.

4. X-ray diagnostic apparatus according to one of the above Claims, characterized in that a high voltage source for the X-ray source (32) is incorporated in the X-ray source module (28) and that a high voltage source for the image intensifier tube (34) is incorporated in the image intensifier tube module (30).

5. X-ray diagnostic apparatus according to one of the above Claims, characterized in that a C-arc module with the actual C-arc (24) and a roller mechanism (22) for this is detachably connected to a pedestal (20) for this by a blockable dovetail joint.

6. X-ray diagnostic apparatus according to one of the above Claims, characterized in that a pedestal (20) for the C-arc (24) together with a rotatable and height-adjustable column (16) which can be mounted on the support (2) is detachable from the support (2) as a module.

7. X-ray diagnostic apparatus according to one of the above Claims, characterized in that an electrical control module (12) is detachably coupled to the support (2).

8. X-ray diagnostic apparatus according to Claim 7, characterized in that a drive mechanism for adjusting the height of the C-arc (24) can at least be operated manually when the electrical control module (12) is removed.

9. X-ray diagnostic apparatus according to one of the above Claims, characterized in that the X-ray source (32) can be rotated across an end face of the C-arc (24) through an angle of between + and - 90° with respect to a position aimed at the image intensifier (34) and is automatically blocked in this latter position.

10. X-ray diagnostic apparatus according to one of the above Claims, characterized in that a leg (8) of the support (2) projecting in the direction of the C-arc (24) is fixed so that it can be detached or swung up.

11. X-ray diagnostic apparatus according to one of the above Claims, characterized in that activation of the X-ray source (32) for a given phase of operation is blocked as long as all the components for that phase of operation are not correctly assembled and electrically connected.

12. X-ray diagnostic apparatus according to Claim 11, characterized in that in a position in which the X-ray source (32) is not aimed at the image intensifier tube (34) activation for fluoroscopy is blocked.

13. X-ray diagnostic apparatus according to one of the above Claims, characterized in that all the dismantling operations are indicated in a logical sequence on the machine with distinguishing marks.

14. X-ray diagnostic apparatus according to Claim 12, characterized in that close to interconnecting surfaces the modules to be coupled are provided with a reference marking in order of the sequence of operations.

15. X-ray diagnostic apparatus according to one of the above Claims, characterized in that all the coupling and uncoupling levers have an alarm colour which differs sharply from other parts of the apparatus.

**Patentansprüche**

1. Diagnostischer Röntgenapparat mit einem mit einer Anzahl von Rädern (6, 10) versehenen verfahrbaren Träger (2), mit einem vom Träger unterstützten C-Bogen (24), mit einer Röntgenquelle (32) und einer an den betreffenden Enden des C-Bogens (24) befestigten Bildverstärkerröhre (34), dadurch gekennzeichnet, daß der Apparat aus abnehmbaren Moduln zusammengesetzt ist, die einen elektrischen Speisemodul (12), einen Röntgenquellenmodul (22) und einen Bildverstärkermodul (30) umfassen, wobei der Röntgenquellenmodul (28) und der Bildverstärkermodul (30) über jeweilige Kopplungen abnehmbar am C-Bogen (24) befestigt sind, die Kopplungen je einen elektrischen Verbinder enthalten, der bei mechanischer Verbindung des Röntgenquellenmoduls (28) und des Bildverstärkermoduls (30) mit dem C-Bogen (24) diese Moduln mit dem elektrischen Speisemodul (12) elektrisch verbindet.

2. Diagnostischer Röntgenapparat nach Anspruch 1, dadurch gekennzeichnet, daß die Kopplungen zum Positionieren des Bildverstärkermoduls (30) und des Röntgenquellenmoduls (28) in bezug auf den C-Bogen (24) mit Pilotstiften ausgerüstet sind.

3. Diagnostischer Röntgenapparat nach Anspruch 2, dadurch gekennzeichnet, daß die elektrischen Verbinder Stecker und Buchsen umfassen, die beim Positionieren gegen den Teil verschiebbar sind, mit dem sie gekoppelt werden, wobei zum Ausüben einer Kraft auf die Stecker und die Buchsen beim Verschieben eine Feder mit den Steckern oder Buchsen verbunden ist.

4. Diagnostischer Röntgenapparat nach einem der vorgenannten Ansprüche, dadurch gekennzeichnet, daß in den Röntgenquellenmodul (28) eine Hochspannungsquelle für die Röntgenquelle (32) aufgenommen ist, und in den Bildverstärkerröhrenmodul (30) eine Hochspannungsquelle für die Bildverstärkerröhre (34) aufgenommen ist.

5. Diagnostischer Röntgenapparat nach einem der vorgenannten Ansprüche, dadurch gekennzeichnet, daß ein C-Bogenmodul mit dem momentanen C-Bogen und einer Rollenvorrichtung (22) dafür mittels einer verriegelbaren Schwalbenschwanzverbindung lösbar mit einem Stand (20) verbunden ist.

6. Diagnostischer Röntgenapparat nach einem der vorgenannten Ansprüche, dadurch gekennzeichnet, daß ein Stand (20) für den C-Bogen (24) zusammen mit einer drehbaren und höheneinstellbaren Säule (16), die auf dem Träger (2) montierbar ist, als Modul vom Träger (2) abnehmbar ist.

7. Diagnostischer Röntgenapparat nach einem der vorgenannten Ansprüche, dadurch gekennzeichnet, daß ein elektrischer Steuermodul (12) mit dem Träger (2) abnehmbar gekoppelt ist.

8. Diagnostischer Röntgenapparat nach Anspruch 7, dadurch gekennzeichnet, daß eine Antriebsvorrichtung zum Einstellen der Höhe des C-Bogens (24) nach Entfernen des elektrischen Steuermoduls (12) wenigstens von Hand bedienbar ist.

9. Diagnostischer Röntgenapparat nach einem der vorgenannten Ansprüche, dadurch gekennzeichnet, daß die Röntgenquelle (32) über eine Endfläche des C-Bogens (24) unter einem Winkel zwischen + und - 90° in bezug auf eine auf den Bildverstärker (34) gerichtete Position drehbar und in dieser Position verriegelbar ist.

10. Diagnostischer Röntgenapparat nach einem der vorgenannten Ansprüche, dadurch gekennzeichnet, daß ein sich in Richtung des C-Bogens (24) erstreckender Schenkel (8) des Trägers (2) derart festgesetzt ist, daß er abgenommen oder hochgeklappt werden kann.

11. Diagnostischer Röntgenapparat nach einem der vorgenannten Ansprüche, dadurch gekennzeichnet, daß Aktivierung der Röntgenquelle (32) in einer vorgegebenen Betriebsphase für die Dauer der noch nicht einwandfreien Montage und elektrischen Verbindung gesperrt ist.

12. Diagnostischer Röntgenapparat nach Anspruch 11, dadurch gekennzeichnet, daß in einer Position, in der die Röntgenquelle (32) nicht auf die Bildverstärkerröhre (34) gerichtet ist, Aktivierung für Fluoroskopie gesperrt ist.

13. Diagnostischer Röntgenapparat nach einem der vorgenannten Ansprüche, dadurch gekennzeichnet, daß alle Abmontierungshandlungen mit Kennmarken in logischer Reihenfolge auf der Maschine verzeichnet sind.

14. Diagnostischer Röntgenapparat nach Anspruch 12, dadurch gekennzeichnet, daß nahe bei den Verbindungsflächen die zu koppelnden Moduln in der Folge der Handlungen mit einer Bezugsmarkierung versehen sind.

15. Diagnostischer Röntgenapparat nach einem der vorgenannten Ansprüche, dadurch gekennzeichnet, daß alle Koppel- und Entkoppelhebel eine Alarmfarbe aufweisen, die sich von den anderen Teilen des Apparats stark unterscheidet.

**Revendications**

1. Appareil de radiodiagnostic comprenant un support mobile (2) comportant plusieurs roues (6, 10), un arc en forme de C (24) supporté par le support, une source de rayons X (32) et un tube amplificateur d'image (34) attaché aux extrémités respectives de l'arc en C (24), caractérisé en ce qu'il est composé de modules détachables comprenant un module d'alimentation électrique (12), un module de source de rayons X (28) et un module d'amplificateur d'image (30), le module de source de rayons X (28) et le module d'amplificateur d'image (30) étant reliés de façon détachable à l'arc en C (24) par des accouplements respectifs, les accouplements comprenant chacun un connecteur électrique qui, lors de la connexion mécanique du module de source de rayons X (28) et du module d'amplificateur d'image (30) à l'arc en C (24), connecte électriquement ces modules au module d'alimentation électrique (12).

2. Appareil de radiodiagnostic suivant la revendication 1, caractérisé en ce que les accouplements sont équipés de broches pilotes pour positionner le module d'amplificateur d'image (30) et le module de source de rayons X (28) par rapport à l'arc en C (24).

3. Appareil de radiodiagnostic suivant la revendication 2, caractérisé en ce que les connecteurs électriques comprennent des fiches et des douilles, les fiches ou les douilles étant, pendant le positionnement, déplaçables par rapport à la partie à laquelle elles sont attachées, un ressort étant relié aux fiches ou aux douilles pour exercer une force sur les fiches et les douilles lors du déplacement.

4. Appareil de radiodiagnostic suivant l'une quelconque des revendications précédentes, caractérisé en ce qu'une source de haute tension pour la source de rayons X (32) est incorporée dans le module de source de rayons X (28) et qu'une source de haute tension pour le tube amplificateur d'image (34) est incorporée dans le module de tube amplificateur d'image (30).

5. Appareil de radiodiagnostic suivant l'une quelconque des revendications précédentes, caractérisé en ce qu'un module d'arc en C comprenant l'arc en C proprement dit (24) et un mécanisme à galets (22) pour ce dernier est relié de façon détachable à une base (20) pour cet arc en C par un assemblage à queue-d'aronde blocable.

6. Appareil de radiodiagnostic suivant l'une quelconque des revendications précédentes, caractérisé en ce qu'une base (20) pour l'arc en C (24) conjointement avec une colonne tournante et réglable en hauteur (16) qui peut être montée sur le support (2) est détachable du support (2) sous forme de module.

7. Appareil de radiodiagnostic suivant l'une quelconque des revendications précédentes, caractérisé en ce qu'un module de commande électrique (12) est couplé de façon détachable au support (2).

8. Appareil de radiodiagnostic suivant la revendication 7, caractérisé en ce qu'un mécanisme d'entraînement pour régler la hauteur de l'arc en C (24) peut au moins être actionné manuellement lorsque le module de commande électrique (12) est enlevé.

9. Appareil de radiodiagnostic suivant l'une quelconque des revendications précédentes, caractérisé en ce que la source de rayons X (32) peut être tournée sur une face d'extrémité de l'arc en C (24) d'un angle compris entre + et -90° par rapport à une position pointée vers l'amplificateur d'image (34) et est bloquée automatiquement dans cette dernière position.

10. Appareil de radiodiagnostic suivant l'une quelconque des revendications précédentes, caractérisé en ce qu'un pied (8) du support (2) s'étendant en direction de l'arc en C (24) est fixé de manière à pouvoir être détaché ou rabattu vers le haut.

11. Appareil de radiodiagnostic suivant l'une quelconque des revendications précédentes, caractérisé en ce que l'activation de la source de rayons X (32) pour une phase de fonctionnement donnée est bloquée aussi longtemps que tous les composants pour cette phase de fonctionnement ne sont pas correctement assemblés et électriquement connectés.

12. Appareil de radiodiagnostic suivant la revendication 11, caractérisé en ce que, dans une position dans laquelle la source de rayons X (32) n'est pas pointée vers le tube amplificateur d'image (34), une activation pour la fluoroscopie est bloquée.

13. Appareil de radiodiagnostic suivant l'une quelconque des revendications précédentes, caractérisé en ce que toutes les opérations de démontage sont indiquées dans un ordre logique sur la machine par des marques distinctives.

14. Appareil de radiodiagnostic suivant la revendication 12, caractérisé en ce qu'à proximité de surfaces d'interconnexion, les modules à coupler sont pourvus d'une marque de référence dans l'ordre de la séquence d'opérations.

15. Appareil de radiodiagnostic suivant l'une quelconque des revendications précédentes, caractérisé en ce que tous les leviers d'accouplement et de désaccouplement ont une teinte d'alarme qui diffère nettement de la teinte des autres parties de l'appareil.

FIG.1

FIG.2

FIG.3